# EUROPEAN PATENT SPECIFICATION

(11) **EP 3 060 199 B1**
(45) Date of publication and mention of the grant of the patent: **01.05.2019**
(21) Application number: 14790261.3
(22) Date of filing: 21.10.2014
(51) Int. Cl.: A61K 9/14, A61P 35/00

(54) **SHIELDED BIOLOGIC THERAPEUTIC**
ABGESCHIRMTES BIOLOGISCHES THERAPEUTIKUM
AGENT THÉRAPEUTIQUE BIOLOGIQUE PROTÉGÉ

(30) Priority: 22.10.2013 GB 201318664
(43) Date of publication of application: 31.08.2016
(73) Proprietor: Oxsonics Limited, Oxford, OX4 4GA (GB)
(72) Inventor: MO, Steven, Oxford Oxfordshire OX3 7DQ (GB); CARLISLE, Robert, Crispin, Oxford Oxfordshire OX3 7DQ (GB); SEYMOUR, Leonard, W., Oxford Oxfordshire OX3 7DQ (GB); COUSSIOS, Constantin-Cassios, Oxford Oxfordshire OX3 7DQ (GB)
(74) Representative: J A Kemp
(86) International application number: PCT/GB2014/053134
(87) International publication number: WO 2015/059459

(56) References cited:
- WO-A1-2009/039502
- WO-A1-2011/072133
- WO-A2-2010/048623
- STEVEN MO ET AL: "Coating of adenovirus type 5 with cleavable PEGylated gold nanoparticles for enhanced protection and circulation", HUMAN GENE THERAPY, MARY ANN LIEBERT, NEW YORK ,NY, US, [Online] vol. 24, no. 5, 1 May 2013 (2013-05-01), XP002719082, ISSN: 1043-0342, DOI: 10.1089/HUM.2013.2505 Retrieved from the Internet: URL:http://online.liebertpub.com/doi/pdf/1 0.1089/hum.2013.2505> [retrieved on 2013-05-06]
- DE-HAO TSAI ET AL: "Tumor necrosis factor interaction with gold nanoparticles", NANOSCALE, vol. 4, no. 10, 1 January 2012 (2012-01-01), page 3208, XP55155725, ISSN: 2040-3364, DOI: 10.1039/c2nr30415e
- MESSERSCHMIDT SYLVIA K E ET AL: "Targeted lipid-coated nanoparticles: Delivery of tumor necrosis factor-functionalized particles to tumor cells", JOURNAL OF CONTROLLED RELEASE, vol. 137, no. 1, July 2009 (2009-07), pages 69-77, XP002733402, ISSN: 0168-3659
- VISARIA RACHANA K ET AL: "Enhancement of tumor thermal therapy using gold nanoparticle-assisted tumor necrosis factor-alpha delivery", MOLECULAR CANCER THERAPEUTICS, vol. 5, no. 4, April 2006 (2006-04), pages 1014-1020, XP002727905, ISSN: 1535-7163
- XIUFANG WANG ET AL: "Assembly of dandelion-like Au/PANI nanocomposites and their application as SERS nanosensors", BIOSENSORS AND BIOELECTRONICS, vol. 26, no. 6, 1 February 2011 (2011-02-01), pages 3063-3067, XP55155843, ISSN: 0956-5663, DOI: 10.1016/j.bios.2010.11.044
- SOLIMAN MAHMOUD ET AL: "Multicomponent Synthetic Polymers with Viral-Mimetic Chemistry for Nucleic Acid Delivery", MOLECULAR PHARMACEUTICS, AMERICAN CHEMICAL SOCIETY, US, vol. 9, no. 1, 1 January 2012 (2012-01-01) , pages 1-13, XP008173697, ISSN: 1543-8384
- MO S ET AL: "Goldnanoparticles for the enhancement of adenovirus stealthing", HUMAN GENE THERAPY, vol. 24, no. 12, December 2013 (2013-12), pages A67-A68, XP002733403, & COLLABORATIVE CONGRESS OF THE EUROPEAN-SOCIETY-FOR-GENE-AND-CELL-THERAPY AND THE SPANISH-SOCIETY-FOR; MADRID, SPAIN; OCTOBER 25 -28, 2013

## Description

### Field of the Invention

The invention disclosed herein relates to the field of biologic medical products (biologic therapeutics), and in particular biologic therapeutics having improved shielding from bloodstream components when *in vivo.*

### Background of the Invention

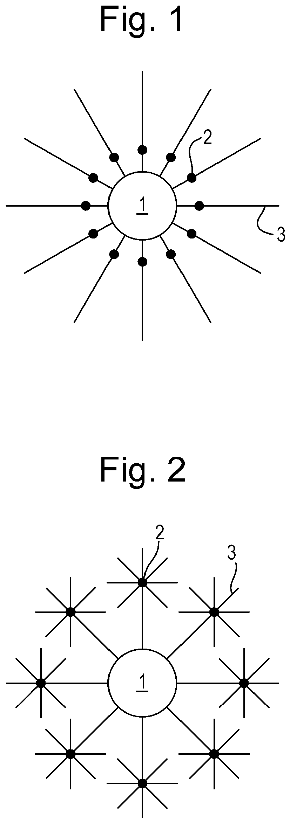

A long half-life of a biologic in systemic circulation is desired in order to improve the therapeutic effectiveness of a biologic. It is known that the interaction of bloodstream components with biologics reduces the circulation half-life of the biologic *in vivo* (the time taken for the number of biologics present in the systemic circulation to reduce by half). Attempts have been made to increase the circulation half-life of systemically administered biologics such as adenovirus type 5 (Ad5) by chemical modification. Such attempts have included covalently attaching non-cleavable or cleavable polymer chains to biologics such as Ad5. Polymers used include monovalent polyethylene glycol (PEG) chains (PEGylation) which form a single attachment to the biologic, or multivalent poly(hydroxypropyl methylacrylamide) (PHPMA) chains which forms multiple linkages to the biologic per polymer. Attempts have also included shielded biologics such as that illustrated in Figure 1 involving insertion of gold particles 2 between the PEG polymer 3 and the biologic 1. Providing a shielded biologic in this way necessitates a large number of points of attachment of polymer to the surface of a biologic, in order to provide the dense steric protection required for the desired shielding effect. However, extensive modification of the surface of a biologic in this way can produce undesirable changes to its structure and activity. There is therefore a trade-off in presently known shielding methodologies between providing dense protection of a biologic, and minimising surface modification.

WO 2010/048623 describes a nanocluster or nanorose composition comprising two or more closely spaced nanoparticles each comprising one or more metals, metal oxides, inorganic substances, or a combination thereof and one or more stabilizers. The stabilizers are in contact with the two or more closely spaced nanoparticles in order to form a nanocluster composition.

### Summary of the Invention

The present invention provides a way to maximise the density of protection of a biologic with minimal surface modification. That is achieved by attachment to the biologic of a plurality of nanoparticles, each nanoparticle having a plurality of polymer modifications (a so-called "dandelion"). In that way dense protection is provided by the plurality of polymer chains 3 attached to the nanoparticle 2, without the need for each polymer chain to be attached directly to the surface of the biologic 1 (one embodiment illustrated schematically in Figure 2). The nanoparticles are bound to the biologic covalently (via a covalently bound linker) and therefore the linkage is stable when the compound is in the vasculature. The nanoparticles are, however, cleavably bound to the biologic, thus the covalent linker between the biologic and nanoparticle can be cleaved under certain conditions.

Thus, the present invention provides a shielded biologic therapeutic comprising a biologic therapeutic which is covalently and cleavably bound to a plurality of nanoparticles, each nanoparticle having a plurality of polymer chains bound thereto, wherein the biologic therapeutic is covalently bound to each nanoparticle via a polymer chain, each polymer chain comprising one or more cleavable moieties.

The present invention also provides a shielded biologic therapeutic as described herein for use in a method of treatment by therapy of a human or animal subject. In some embodiments the method further comprises exposing the subject to ultrasound-induced cavitation. The method may be for the treatment of a tumour.

The present invention also provides a method of diagnosis of the human or animal body which comprises administering a shielded biologic therapeutic as described herein. Preferably, the method further comprises exposing the subject to ultrasound-induced cavitation. The method may be for the diagnosis of a tumour.

Also described herein is a non-toxic, biocompatible polymer-modified nanoparticle comprising a nanoparticle having a plurality of polymer chains bound thereto. Said nanoparticle having a plurality of polymer chains bound thereto is typically suitable for use as a shielding component in a shielded biologic therapeutic.

The identity of the biologic therapeutic is not particularly limited, and the shielded biologic therapeutic of the present invention can comprise any biologic which may be desired for a particular therapeutic purpose. Biologics themselves are well known, and are known to be capable of surface modification. A skilled person will understand that the polymer modified nanoparticle or "dandelion" described herein can be cleavably bound to any biologic therapeutic.

Similarly, the identity of the nanoparticle is not particularly limited. Any nanoparticle amenable to surface modification can be used in the shielded biologic therapeutic of the invention. The nanoparticle used in the invention typically has low-toxicity and good biocompatibility. However, in some embodiments a nanoparticle may be used which itself has sub-optimal toxicity and biocompatibility, but when a plurality of polymer chains are attached thereto the polymer-modified nanoparticle (i.e. the nanoparticle and polymer chains taken together) has low-toxicity and good biocompatibility.

### Brief description of the Figures

Figure 1 illustrates a prior art polymer-modified biologic
Figure 2 illustrates one embodiment of the shielded biologic therapeutic of the present invention.
Figures 3a and 3b show zeta potential of each gold and Ad conjugation step was measured (n = 5, SD shown); *, **, and *** represents p-value < 0.05, 0.01, and 0.001, respectively.
Figure 3c shows gel electrophoresis showing reduction-reversible retarded migration of Ad proteins following conjugation to gold-PEG. SDS-PAGE silver staining was performed + or - 'BME' reducing buffer (50 mM beta-mercaptoethanol), lanes 1 and 2 = Ad, 3 and 4 = Ad-gold-PEG, 5 and 6 = Ad + gold-PEG. Roman numerals denote positions of Ad proteins according to molecular weight.
Figure 3d shows transmission electron microscopy of gold-PEG, Ad, Ad + gold-PEG or Ad-gold-PEG constructs. The red scale bar represents 50 nm. For fixation, visualisation, and image capture, see on line materials and methods.
Figure 4a shows results of an ELISA. Ad = non-modified Ad, Ad + gold-PEG = control non-linked Ad and gold-PEG, Ad-gold-PEG = chemically conjugated Ad and gold-PEG. N = 5, SD shown.
Figure 4b shows expression of the GFP transgene encoded by the Ad, in terms of % of cells positive for GFP.
Figure 4c shows mean fluorescence intensity (MFI) per positive cell. N = 4, SD shown.
Figure 4d shows the influence of gold-PEG modification on protection of FX binding domains on Ad hexon is shown by performing infections in the presence and absence of FX and BME, N = 4, SD shown.
Figure 5a shows blood sampling and quantification by QPCR. n = 4, S.D shown. Ad-gold-PEG, different from all other groups.
Figure 5b shows total percentage of the injected dose accumulated in livers.
Figure 5c shows total percentage of dose accumulated per gram of tumour mass. Each group had four mice (n = 4), standard deviation shown. Groups compared using ANOVA followed by Newman-Keuls test for pairwise comparison of sub-groups; * and *** represents p-value < 0.05 and 0.001, respectively.
Figure 5d shows the relationship between Ad plasma circulation profile and tumor accumulation. Each point represents one mouse treated with Ad (black square), Ad-PEG (white triangle), Ad-PHPMA (purple circle), and Ad-gold-PEG (blue triangle). Area under curve calculated from circulation data at 30 min time point for all mice, N=8. Correlation between AUC and Ad tumor accumulation (R² = 0.6968) .
Figures 6a and b show in vitro ultrasound set-up
Figures 6c-f show influence of ultrasound exposure pressure on the penetration of Ad samples into TMM as assessed by QPCR. For each figure the left panel shows the number of Ad recovered at different depths from the vessel N = 4, SD shown, ANOVA analysis and the right panel shows a representative frequency spectra detected over the course of the ultrasound exposure. Passive cavitation detection, shows increasing broadband acoustic emissions with increasing pressure of exposure, indicative of the occurrence of inertial cavitation.
Figure 6g shows fluorescence microscopy analysis of green fluorescent protein transgene production from Ad or Ad-gold-PEG after ultrasound exposure at 180 or 1250 kPa, with or without BME treatment and 24 hours incubation. White dotted lines denote flow channel, as in Fig 6b, and white arrows demarcate extent of infected region.
Figures 7a and 7b show the influence of ultrasound on active targeting to tumors. Fig 7a shows biodistributiuon and Fig 7b shows tumor accumulation at 30 min using QPCR, n =4, SD shown, ANOVA analysis used.

### Detailed Description of the Invention

As used herein a biologic therapeutic refers to any biological material suitable for a therapeutic or *in vivo* diagnostic purpose. Biologic therapeutics include peptides, proteins, vaccines, antibodies, aptamers, nucleic acids, DNA, RNA, antisense oligonucleotides, viruses and bacteria.

As used herein a nanoparticle is any nano-scale particle, typically from 1 to 1000 nanometres in size.

As used herein the terms "non-toxic and biocompatible" refer to the ability of a component of a shielded biologic therapeutic to perform its desired function with respect to a medical therapy or method of diagnosis without eliciting any therapeutically unacceptable local or systemic effects in the recipient or beneficiary of that therapy or diagnosis.

As used herein the terms "cleavable" and "covalently and cleavably bound", refer to a covalent linkage which is stable under certain conditions, e.g. stable when in the vasculature but cleavable under certain other conditions. Covalent linkages may for example be cleavable under certain pH conditions, under reducing conditions or oxidising conditions, or they may be cleavable in the presence of particular enzymes, e.g. under enhanced levels of organ-specific endopeptidases (e.g. matrix metalloproteinases (MMP2)). Covalent, cleavable linkages used in the present invention involve linkage of the nanoparticle to the biologic via a polymeric linker, which is itself covalently bound to both the biologic and the nanoparticle. Cleavage may occur at any position in the linker including at the bond to the nanoparticle, the bond to the biologic, or elsewhere in the linker moiety.

As used herein a shielding component is the nanoparticle having a plurality of polymer chains bound thereto which forms part of the shielded biologic therapeutic described herein. A nanoparticle having a plurality of polymer chains bound thereto will typically be suitable for use as a shielding component in the shielded biologic therapeutic described herein if it is non-toxic and biocompatible, and if it comprises a moiety capable of being cleavably bound to a biologic therapeutic.

As used herein, the term "shielded" means sterically protected. Thus, a shielded biologic therapeutic is a biologic therapeutic molecule having a surface which is sterically protected. Such shielded molecules are less susceptible to reaction *in vivo,* and accordingly have an increased in vivo half life. The increase in half life is achieved by virtue of the physical coverage of the surface of the molecule with shielding components. A measure of the degree of modification of a biologic therapeutic is the proportion of surface amine groups that are removed. In the present invention, typically at least 5%, e.g. at least 7%, 8% or 10%, of the surface amine groups are modified. The amount of shielding such modification provides can be assessed by an enzyme-linked immuno-sorbant assay (ELISA) with comparison to conventional coating strategies such as pegylation. The in vivo half life of the biologic is the ultimate measure of the degree of shielding.

As used herein the term "dandelion" refers to a nanoparticle having a plurality of polymer chains bound thereto. The dandelion may refer to part of a shielded biological therapeutic, or may refer to a nanoparticle having a plurality of polymer chains bound thereto as a separate entity which is suitable for use as a shielding component in a shielded biologic.

As described above, the biologic therapeutic is not particularly limited. Particular biologic therapeutics include but are not limited to oncolytic viruses such as Ad5. Ad5 may be used for example when the intended therapeutic use of the biologic therapeutic is as an antitumour agent, due to its high infection efficiency. Ad5 is capable of surface modification, for example at the Ad capsid protein.

Particular nanoparticles include but are not limited to metals such as gold, magnetic particles such as iron oxide, quantum dots or ultrasound responsive carbon nanoparticles. Gold may be preferred for certain therapeutic purposes because of its low toxicity, biocompatibility, suitability for surface modification and high density. The high density of gold means that the density of biologic therapeutics having gold as the nanoparticle is increased. Shielded biologic therapeutics comprising a high density nanoparticle are preferred for therapeutic applications which involve inertial cavitation. Inertial cavitation techniques used in combination with shielded biologic therapeutics comprising a high density nanoparticle are described in greater detail further below. Nanoparticles which facilitate initiation of cavitation are also preferred for therapeutic applications involving inertial cavitation, and include carbon nanoparticles.

Other cavitation initiators which may be used in the shielded biologic therapeutic of the invention include known cavitation inducing nanoparticles, such as those described in Mo et al.; Expert Opin Drug Deliv; 2012; 9(12); 1525-38 and WO 2012/066334.

Quantum dots and/or magnetic nanoparticles such as iron oxide may be preferred in therapeutic applications requiring imaging.

The size of each nanoparticle is typically from 1 to 1000 nanometres, e.g 1 to 500, 1 to 100 or 1 to 10 nanometres. The size of the nanoparticle used will depend on the desired overall size of the shielded biologic therapeutic, which will in turn depend on the target of the biologic therapeutic as discussed in more detail further below.

The polymer chains bound to each nanoparticle are typically non-toxic and biocompatible, e.g. a non-toxic, biocompatible hydrophilic polymer. Particular polymer chains which may be bound to each nanoparticle include but are not limited to poly (alkylene oxides), e.g. PEG, and PHMPA. PEG is preferred.

Methods of attaching polymer chains to a nanoparticle are known in the art and include, for example, carbodiimide (EDG) chemistry which is suitable for attaching PEG polymer chains to nanoparticles including gold. Polymer chains can also be attached to nanoparticles using reactions between N-hydroxysuccinimide or thiazolidine-2-thione groups and amine groups or between maleimide and thiol groups.

The dandelion typically comprises a plurality of polymer chains each having a molecular weight MW1 and one or more polymer chains having a molecular weight MW2, wherein MW2 is greater than MW1 and the number of polymer chains having molecular weight MW1 is greater than the number of chains having MW2. Thus, the dandelion typically comprises a relatively high number of relatively short polymer chains, and one or a relatively low number of relatively long polymer chains. MW1 and MW2 may each independently represent a particular molecular weight, or may represent a distribution of molecular weights.

For example, 75% to 99.99% of the polymer chains attached to the dandelion may be of MW1 and 25% to 0.01% of the polymer chains attached to the dandelion may be of MW2. Typically, 90% to 99.9% of the polymer chains attached to the dandelion are of MW1 and 10% to 0.1% are of MW2. Preferably, 95% to 99.5% of the polymer chains are of MW1 and 5% to 0.5% are of MW2. In one embodiment 97.5% to 98.5% of the polymer chains are of MW1 and 2.5% to 1.5% of the polymer chains are of MW2. The strategy applies the ratios of MW1 and MW2 most appropriate to provide optimal modification of the particular nanoparticle to allow protection of the particular biologic with minimal modification to its surface.

The molecular weight of the polymer chains will depend on the desired overall size of the shielded biologic therapeutic, which will in turn depend on the target of the biologic therapeutic as discussed in more detail further below. Typically, MW1 is from 1 to 3 kD. Typically, MW2 is from 4 to 30 kD, e.g. 4 to 6kD. Preferably, MW1 is from 1.5kD to 2.5kD. Preferably, MW2 is from 4.5kD to 5.5kD. In one embodiment, MW1 is 2kD and MW2 is 5kD.

In a preferred embodiment 99% of the polymer chains in the dandelion have a molecular weight of 2kD and 1% of the polymer chains in the dandelion have a molecular weight of 5 kD.

Typically up to 99%, e.g 1% to 99% of the surface of each nanoparticle is modified by attachment to a polymer chain. Typically at least 50% of the surface of each nanoparticle is modified by attachment to a polymer chain. In some embodiments 50% to 99% of the surface of the nanoparticle is modified by attachment to a polymer chain, e.g. 80% to 99%, 85% to 95%, 88% to 92% or about 90%.

The number of polymer chains attached to each nanoparticle will depend on the size of the nanoparticle and the surface available for modification. In some embodiments the number of polymer chains attached to each nanoparticle is three or more. For a nanoparticle of 1-10 nm in size, 100 to 500 polymer chains are typically attached to each nanoparticle, e.g. 200 to 300 polymer chains.

The cleavable linkage in the shielded biologic therapeutic is typically formed by one or more (e.g. 1, 2, 3, 4, or 5) of the polymer chains in the dandelion. Preferably, the cleavable linkage is formed by one or more polymer chains in the dandelion having MW2, i.e. one or more of the longer polymer chains. The polymer chains forming the cleavable linkage typically comprise a cleavable moiety. The cleavable moiety typically forms a point of attachment between the biologic therapeutic and a polymer chain of the dandelion.

The presence of a polymer chain as the covalent, cleavable linkage provides greater distance between the nanoparticle and the biologic. This is turn enables larger dandelions (e.g. having more, or longer, polymer chains attached thereto) to be used. Improved shielding is therefore achieved by use of a polymeric chain as a linker.

The cleavable moiety is typically designed to be cleaved under conditions present in the target of the biologic therapeutic, in order to present the free biologic. For example, if the target of the biologic therapeutic is tumour tissue where reducing conditions prevail, the cleavable moiety may be cleavable under reducing conditions. Known moieties which are cleavable under reducing conditions include moieties comprising a S-S bond, such as that achieved using the crosslinker N-succinimidyl-3-(2-pyridyldithio)propionate (SPDP). Other moieties which are cleavable under certain conditions including for example a pH below 7.365 or the presence of organ specific endopeptidases, are known in the art. For example, acid labile hydrazide bonds are cleavable under reduced pH conditions and peptide bonds may be cleaved by endopeptidases (e.g. matrix metalloproteinase (MMP2)). A skilled person can therefore select an appropriate cleavable moiety for a shielded biologic therapeutic designed to target tissue having for example oxidising conditions, basic conditions, acidic conditions, conditions with raised endopeptidase levels (e.g. MMP2).

Methods for attaching a biologic therapeutic to a nanoparticle via a polymer chain comprising a cleavable moiety are also known in the art and include the use of bifunctional crosslinking agents such as SPDP which is comprised of a N-hydroxysuccinimide ester to provide reactivity to primary amine groups and a 2-pyridyldithio to provide reactivity to sulphydryl groups.

The shielded biologic therapeutic of the invention comprises one or more dandelions. The number of dandelions required to provide adequate shielding will depend of on the size of the biologic therapeutic. For example, for when the biologic therapeutic is a virus of around 140nm, it may be attached to up to 500 dandelions, 10-300 dandelions or 50 to 200 dandelions. In one embodiment the shielded biologic therapeutic comprises 80 to 120 dandelions. However, when the biologic therapeutic is smaller, for example an antibody, the number of dandelions present may be up to 10, for example 2-5 or 2. A plurality of dandelions are present. Based on the examples above, and knowing the size of the biologic therapeutic requiring shielding, a skilled person can select an appropriate number of dandelions to attach without undue burden.

The number of dandelions present in the shielded biologic therapeutic will also depend on the desired overall size of the shielded biologic therapeutic, which will in turn depend on the target of the biologic therapeutic as discussed in more detail further below.

As mentioned above, the overall size of the shielded biologic therapeutic is dependent on the length (molecular weight) of the polymer chains used, the number of polymer chains attached to each nanoparticle, the size of each nanoparticle, the number of dandelions attached to the biologic therapeutic and size of the biologic therapeutic itself. A skilled person, having in mind a particular biological target may have a desired size of a shielded biologic therapeutic for a particular therapeutic purpose. Further, having a particular biologic therapeutic in mind and a desired size of a shielded biological therapeutic, a skilled person can select without undue burden polymer chains of appropriate length, an appropriate number of polymer chains to attach to each nanoparticle, an appropriate size of nanoparticle and an appropriate number of dandelions to attach to the biologic therapeutic in order to arrive at the desired overall size.

The overall size of an agent of the invention can be in the region of 100-1000 nm, e.g. 100-500 nm or 100-300 nm.

For example, if a skilled person intends to target a tumour, then a particular size of the agent may be desired in order to improve accumulation in tumour tissue by the enhanced permeability and retention (EPR) effect. Tumour tissues may contain neovasculature having abnormal form and architecture, leading to abnormal molecular and fluid transport dynamics. That can cause agents of around 100 to 500 nm, e.g. 100 to 300 nm in size to accumulate in tumour tissue much more than they do in normal tissues. Agent sizes of 100 to 500 nm, e.g. 100 to 300 nm may therefore be desired for use in methods of treating a tumour disorder.

For example, if the biologic therapeutic is an Ad5 virus of around 140nm in size, and the target is a tumour, then an overall size in the desired range, e.g 100 to 500 nm or around 300 nm can be achieved by attaching 80 to 120, e.g. about 100 dandelions each having a nanoparticle of 5-10 nm, e.g. about 7 nm in size, 400 to 600, e.g about 500 polymer chains of 1.5kD to 2.5kD, e.g. about 2kD in size and 2 to 10 polymer chains of molecular weight 4kD to 6kD , e.g. about 5kD.

A particular density of the shielded biologic therapeutic may be desired to complement the use of mechanical agitation, for example by ultrasound-induced cavitation, in a method of treatment or diagnosis for which the shielded biologic therapeutic is intended. Thus, increasing the density of a diagnostic or therapeutic agent facilitates its delivery and transport when a fluid in which it is suspended is agitated, for example by ultrasound induced inertial cavitation. Inertial cavitation occurs when a void or bubble in the body expands and then rapidly collapses, for example under the influence of ultrasound, causing a shockwave. The shockwaves caused by inertial cavitation or other mechanical agitation can be used to deliver biologic therapeutics to their biological targets *in vivo,* for example by extravasation of a biologic therapeutic from the bloodstream into surrounding tissue.

The density of the shielded biological therapeutic can be tuned by selecting the material, size and number of the nanoparticles present. For example, when each nanoparticle has a density greater than that of the biologic therapeutic, the response of the shielded biologic therapeutic to ultrasound-induced cavitation is increased.

The nanoparticle typically has a density which is greater than that of the therapeutic or diagnostic component, thereby forming an agent which has an overall density greater than if the therapeutic or diagnostic component were administered on its own. The density of the nanoparticle is typically two times or more that of the therapeutic or diagnostic component, e.g. 2.5 times or more, 3 times or more, 3.5 times or more, 4 times or more, 4.5 times or more, or 5 times or more.

The nanoparticle typically has a density of 3 g/mL or more, e.g. 4 g/mL or more, 5 g/mL or more, 10 g/mL or more or 15g/mL or more.

The overall density of the shielded biologic therapeutic is typically 1.5 times or more that of the biologic therapeutic alone, e.g. 2 times or more, 2.5 times or more, 3 times or more, 3.5 times or more or 4 times or more.

The overall density of the agent is typically 1.5 g/mL or more, e.g. 1.75 g/mL or more, 2 g/mL or more, 2.25 g/mL or more, 3 g/mL or more, 3.25 g/mL or more, or 3.5 g/mL or more.

The shielded biologic therapeutic can also be used to complement the use of ultrasound induced cavitation by selecting the identity of the nanoparticle so that it acts as a cavitation initiator. Using cavitation initiating nanoparticles ensures that a cavitation initiator is in the same location as the biologic therapeutic (co-location). Co-location of cavitation initiator and therapeutic or diagnostic substance enhances the effectiveness of the ultrasound induced cavitation technique in delivery and transport of the biologic therapeutic.

In some embodiments, each nanoparticle is an agglomerate of carbon nanoparticles. Voids between carbon nanoparticles in the agglomerate act as bubbles when subjected to ultrasound, expanding and then rapidly collapsing. However, the bubbles in the agglomerate are not destroyed in the process. An agglomerate of carbon nanoparticles typically has an overall size of 10 to 400 nm, e.g. 100 - 300 nm or about 200 nm.

Other suitable cavitation initiators either forming the dense component or provided as a further separate agent include known cavitation inducing nanoparticles, such as those described in Mo et al.; Expert Opin Drug Deliv; 2012; 9(12); 1525-38, and nanoscale particles having spherical or part spherical surface features or surface depressions of from 5 to 50 nm in size as described in WO 2012/066334.

The shielded biologic therapeutics of the invention may be administered by any suitable route, depending on the nature of the method of treatment, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.), transdermally (e.g. by application of a patch, gel or implant) or by inhalation (as a dry powder, a solution, a dispersion, etc).

An amount of the shielded biologic therapeutic to be administered as part of a method of treatment or diagnosis will depend on, for example, the identity of the therapeutic or diagnostic component and can be determined by one of skill in the art. Thus, the dose of the agent of the shielded biologic therapeutic will typically be equivalent to or less than the dose of the biologic therapeutic if administered alone, i.e. the amount of biologic therapeutic present in the shielded biologic therapeutic administered will typically be the same or less than the amount that would be administered if in free form. The dose of the shielded biologic therapeutic of the invention may be less than the equivalent amount of free biologic therapeutic for example to compensate for the enhanced pharmacokinetics seen in the shielded biologic therapeutic of the invention as described above, for example 95% or less, 85% or less, 80% or less, 75% or less, 70% or less, 65% or less, 60% or less, 55% or less, or 50% or less.

### Examples

The invention is illustrated below with reference to certain non-limiting examples. In particular, although the invention is illustrated using a particular biologic therapeutic, a skilled person would understand that the shielding methodology is generally applicable to any biologic therapeutic and would be able to adapt the examples below as appropriate based on the foregoing description.

### Example 1: Formulation and analysis of gold-PEG dandelion and Ad-gold-PEG shielded biologic therapeutic

Carbodiimide (EDC) chemistry was used to attach 5 molecules of 5kDa thiol-PEG of per gold nanoparticle to which a further 257 copies of 2 kDa PEG were added. N-succinimidyl 3-(2-pyridyldithio)propionate (SPDP) was then used to achieve linkage of this highly stealthed construct to Ad via a single reduction-cleavable bond between a 5kDa PEG and an amine groups on the surface of the Ad, to give Ad-gold-PEG

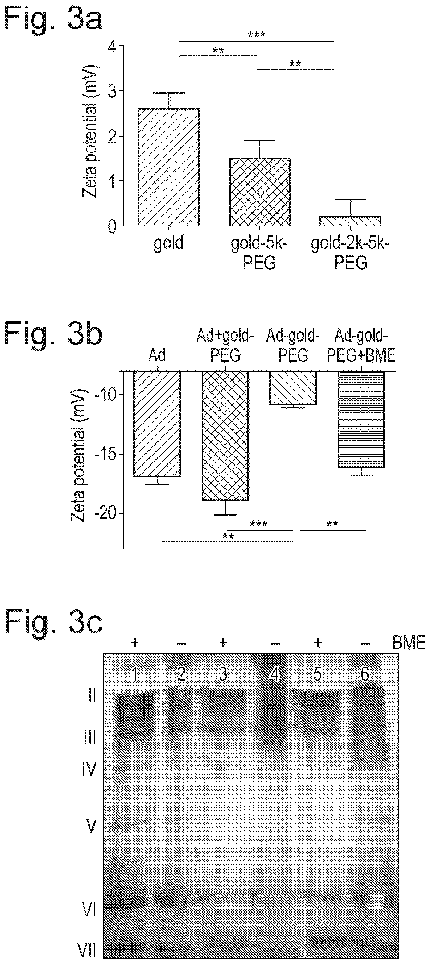

Results from ζ-potential (Fig. 3b) demonstrated that the gold was coated successfully in the dandelions since ζ-potentials became less positive as amine groups on the gold were removed by reaction with PEG, changing from 2.6 to 1.5 mV upon the addition of 5kDa PEG and 0.2 mV after subsequent addition of 2kDa PEG. Figure 3c demonstrates that the dandelions were successfully attached to the Ad as the ζ-potential of Ad increased from - 16.9 to -13.9 mV upon reaction with gold-PEG.

Gold-PEG had a greater hydrodynamic diameter (15 nm) than gold, which measured 6.3 nm. Unmodified Ad measured 117 nm, whereas Ad-gold-PEG measured 149 nm, a 32-nm increase which corresponds to the combined size of two gold-PEG dandelions, demonstrating a good gold-PEG coating geometry.

Treatment of Ad-gold-PEG with reducing agent (beta-mercaptoethanol) cleaved the 5kDa PEG and returned Ad to its original size.

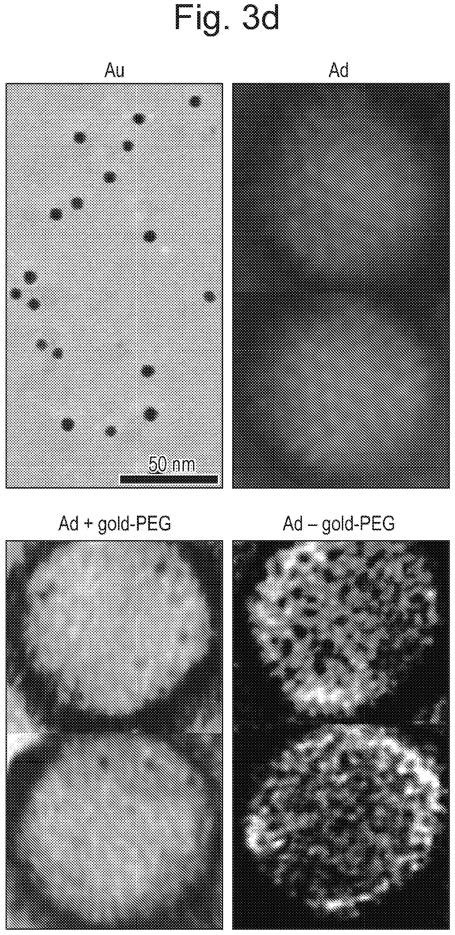

Alteration to Ad capsid protein composition and size after stealthing with gold-PEG was characterized by separating the capsid proteins on a polyacrylamide gel. The resulting SDS-PAGE silver stain (Fig. 3d) indicated that neither Ad (lanes 1 and 2) nor non-linked Ad + gold-PEG (lanes 5 and 6) showed a difference in Ad capsid polypeptide band intensity in the presence or absence of the reducing buffer BME. In contrast, analysis of conjugated Ad-gold-PEG (lanes 3 and 4) showed a dramatically different band migration pattern depending on the presence or absence of reducing buffer. Notably, in the absence of reducing agent (lane 4) there was little discernible migration of Ad capsid protein into the gel, indicating that most Ad capsid protein was bound to gold-PEG and unable to properly penetrate the polyacrylamide. No bands were evident for Ad polypeptides II, III, and IV; notably the bands which did stain in lane 4 corresponded to internal capsid proteins such as VI and VII. However, upon exposure to reducing buffer 9 (lane 3), Ad-gold-PEG showed equivalent protein migration and intensity to that of Ad and non-linked Ad + gold-PEG, signifying the reduction-induced breakage of disulfide bonds between Ad and gold-PEG to un-stealth Ad to its original form. TEM images (Fig. 3e) showed 60 gold-PEG linked per Ad capsid. Notably, because the 12 trimeric fibre proteins are lost from Ad during TEM processing the gold-PEG attached to these regions cannot be visualised by this method. However, as SDSPAGE demonstrated that sufficient gold-PEG was attached to the trimeric fibre proteins to prevent its migration it is reasonable to calculate that at least 3 gold-PEG were attached per fibre. Adding the capsid (60) and fibre values (36) gives a total of approximately 96 gold-PEG per Ad. TNBS analysis showed the loss of 111 amine groups from Ad upon reaction with gold-PEG. These analyses therefore prove that this stealthing procedure enables the overwhelming majority of each of 96 gold-PEG to be linked to Ad by just one bridging 5kDa PEG molecule.

### Example 2: Shielding of Ad-gold-PEG

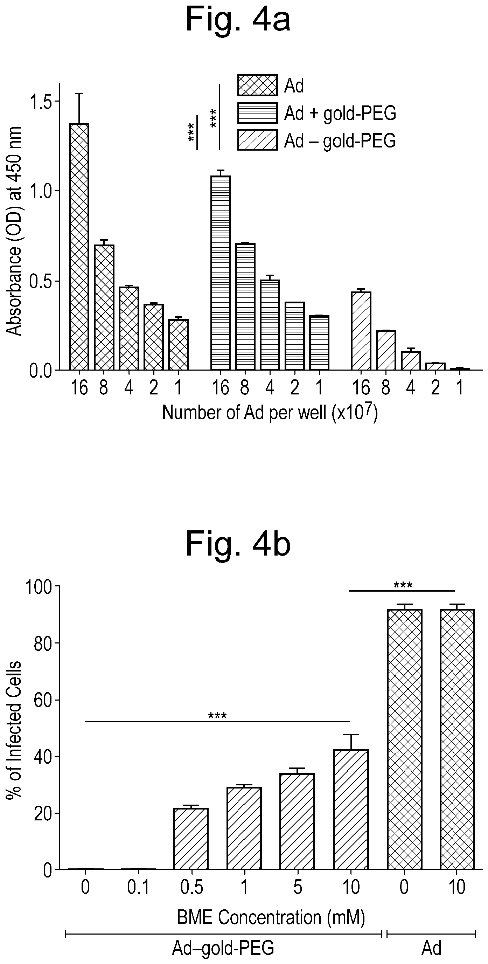

The biological consequences of the changes detected by the physicochemical analyses described in Example 1, were assayed using ELISA and infection of cancer cell lines. ELISA using a polyclonal anti-Ad antibody demonstrated dramatically decreased antibody binding to Ad-gold-PEG compared to Ad and non-linked Ad + gold-PEG (Fig. 4a). Analysis was by one way ANOVA, *** = all groups p<0.001. The utility of the reduction sensitive cleavage and un-stealthing mechanism was demonstrated by infecting with Ad or Ad-gold-PEG which had been pre-incubated with a range of concentrations of the reducing agent BME.

This efficient stealthing was confirmed in studies which showed Ad-gold-PEG to have >10-fold lower (p<0.001) binding to human blood cells than Ad, indicating good protection from complement and antibody mediated sequestration of A-gold-PEG by erythrocytes and leukocytes.

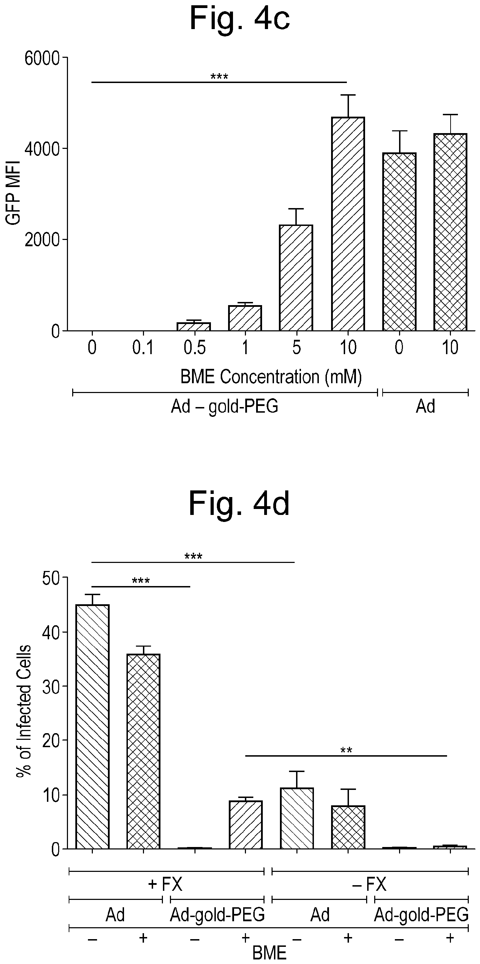

Studies in IGROV-1 cells (which express high levels of the Coxsackie and Adenovirus Receptor - CAR) showed dramatically reduced infection activity for Ad-gold-PEG, indicative that good stealthing of the Ad fiber domain which binds to CAR had been achieved (Figs. 4b and 4c). Furthermore, the utility of the disulfide bond was confirmed as 42% of the infectivity of Ad-gold-PEG could be reactivated upon 20 min exposure to 10 mM BME at a reducing potential matching that of the extracellular tumor milieu. BME treatment provided complete removal of gold-PEG, thereby returning the Ad-gold-PEG to the size and charge of non-modfied Ad (as demonstrated in Example 1).

Studies performed in SKOV-3 cells, which are low in CAR and dependent on Factor X (FX) for infection, indicated acidic regions within hexon were also effectively stealthed, as no FX dependent infection was observed for Ad-gold-PEG (Fig 4d). This is a crucial aspect when attempting to improve circulation of Ad in pre-clinical models.

### Comparative Example 1

Conventionally stealthed Ad-PEG and Ad-PHPMA were prepared to allow comparison to these alternative accepted strategies. Sizes and zeta potentials were measured as in Example 1 to ensure the chemical conjugations were achieved successfully. Ad, Ad-PEG, and Ad-PHPMA were analysed by ELISA, as in Example 2 and results were compared. Ad-gold-PEG demonstrated superior stealthing against the binding of polyclonal anti-Ad antibodies compared to Ad, Ad-PEG, and Ad-PHPMA.

### Example 3: Passive targeting of Ad-gold-PEG to tumors

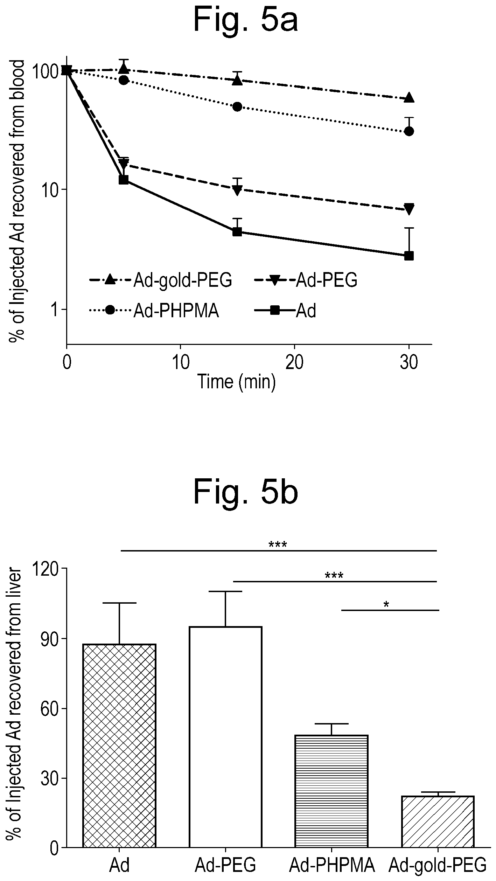

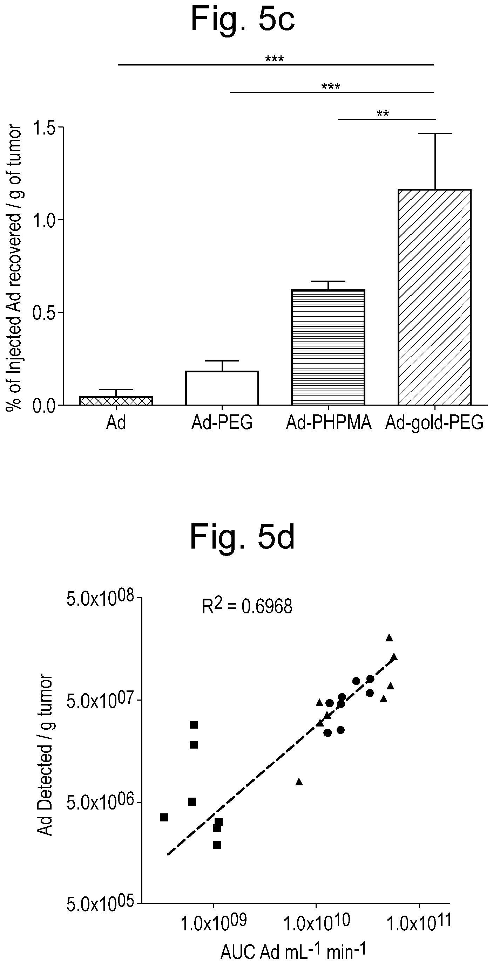

*In vivo* studies were performed in tumor-bearing murine models. After i.v. injection of Ad, Ad-PEG, Ad-PHPMA or Ad-gold-PEG, blood samples were taken at 5, 15, and 30 min, and tumour and liver samples were extracted following cull at 35 min. Blood circulation profiles of Ad, Ad-PEG, Ad-PHPMA and Ad-gold-PEG are shown in Fig. 5.

The control Ad, Ad-PEG and Ad-PHPMA circulation data was comparable to previous published results. The half-life of Ad-gold-PEG was more than 30 min, meaning it outperformed all other groups, including Ad-PHPMA. This indicates that the superior stealthing achieved with Ad-gold-PEG, as demonstrated *in vitro* by ELISA, impacted directly on circulation and hepatic capture *in vivo.* Crucially, TNBS analysis had shown improved stealthing with Ad-gold-PEG was achieved with modification of just 111 capsid amine groups compared to 1332 with Ad-PHPMA or 1007 with Ad-PEG.

Bio-distribution of Ad, Ad-PEG, Ad-PHPMA, and Ad-gold-PEG is represented in Fig. 5b (liver capture) and Fig. 5c (tumor accumulation). More than 90% of Ad and Ad-PEG was captured by the liver. In contrast liver capture of Ad-PHPMA and Ad-gold-PEG decreased to 48% and 21%, respectively. Furthermore, 9-fold more Ad-gold-PEG than Ad particles were recovered from the tumor. Integration of the areas under the curve (AUC) for each sample in Fig. 5c and plotting of these data with their respective total Ad accumulated per gram of tumor, produced a strong correlation (Fig. 5d) with R² = 0.6968, indicating that passive tumor targeting of Ad is dependent on its plasma AUC. This demonstrates that the enhanced chemical coating and protection of Ad-gold-PEG leads to lower liver capture and extended circulation and ultimately EPR assisted increases in passive tumor accumulation.

### Example 3a: Active targeting of Ad-gold-PEG using focussed ultrasound in vitro

Experiments were performed to test if the presence of gold-PEG could increase Ad response to focussed ultrasound and consequently provide improved active delivery to tumors.

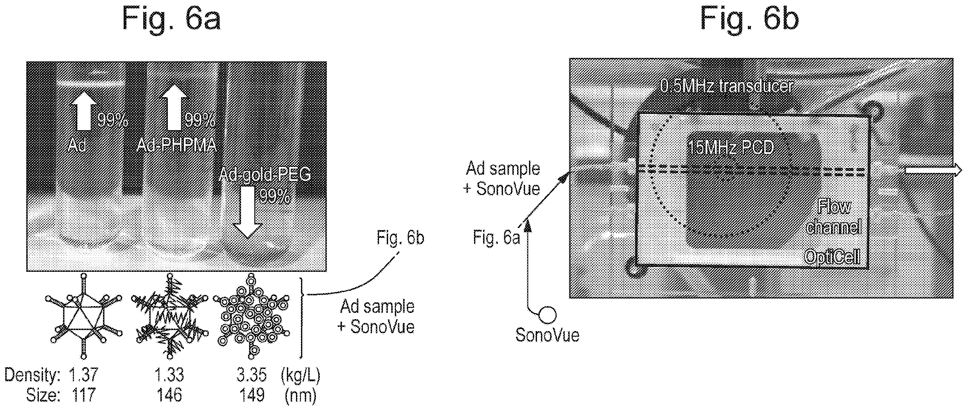

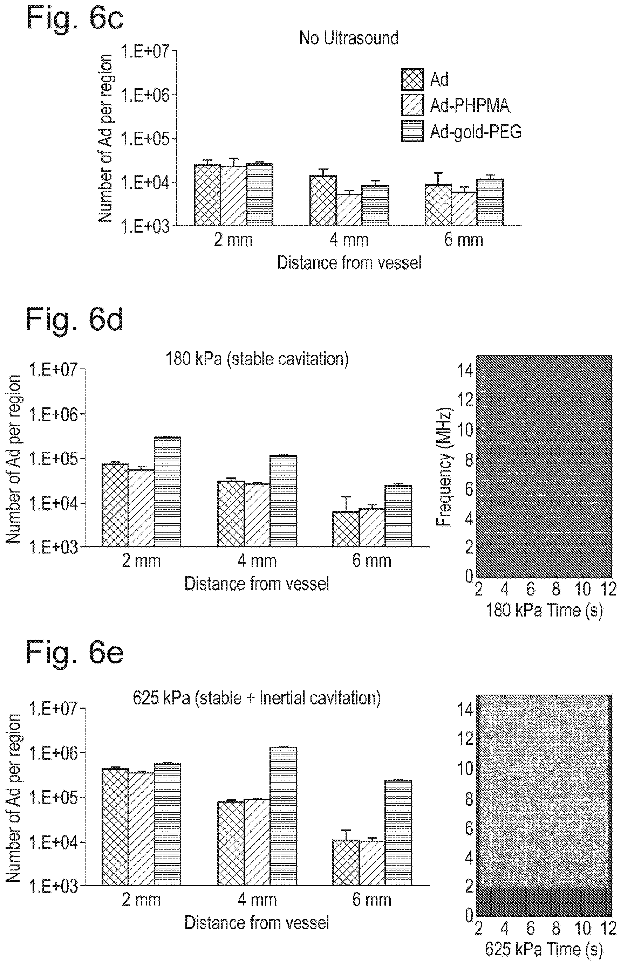

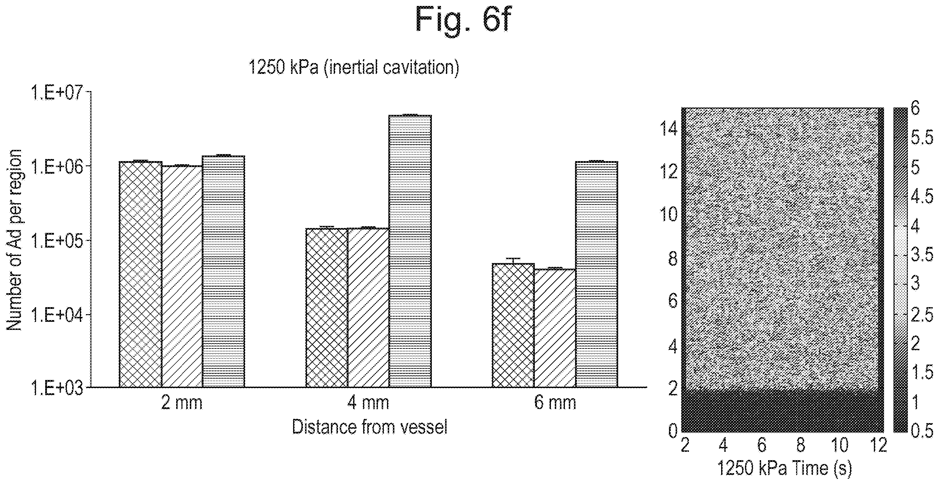

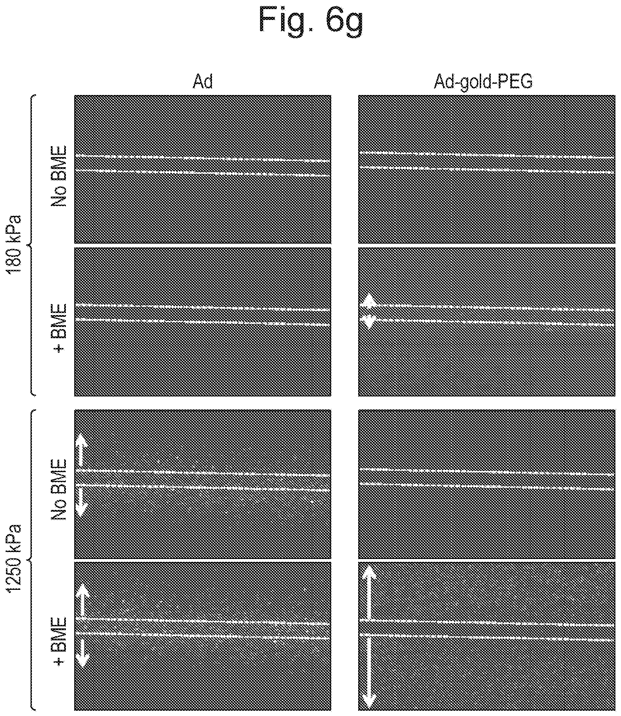

Increasing the density of a nanomedicine such as Ad by its attachment to gold-PEG increased its response to ultrasound induced cavitation events (Fig. 6) when co-injected with cavitation-inducing microbubbles (SonoVue).

The theoretical increase in density in going from Ad (1.37 g/mL) to Ad-gold-PEG (3.35 g/mL) was confirmed by dramatically different ultra-centrifugation separation on caesium chloride gradients of Ad, Ad-PHPMA and Ad-gold-PEG (Fig. 6a). 99% of Ad-gold-PEG being recovered from the bottom of the tube.

When applied through a flow channel in a tissue mimicking material (TMM) and exposed to ultrasound the amount of movement into the TMM (as measured by QPCR for Ad genomes) scaled with the amount of ultrasound induced inertial cavitation events (as measured by passive cavitation detection.

Modulating density altered response to ultrasound and provided precise control over the depth of penetration, which has important implications for the delivery of nanomedicines to tumors as well as transdermally in vaccination procedures. Significantly more Ad-gold-PEG, than Ad or Ad-PHPMA was moved into the TMM at all penetration depths tested. At the maximum pressure tested (1250 kPa), between 50 and 100-fold more Ad-gold-PEG was recovered at distances of 4 and 6 mm from the flow channel. Exposure to BME and analysis of the cells within the TMM for GFP transgene expression at 24 hours confirmed the Ad-gold-PEG to have maintained infection capacity and to have journeyed further than the Ad, whilst also demonstrating that the ultrasound parameters caused no intrinsic cell damage. When quantified using imageJ software significant increase (p<0.001) in the depth of infection was observed. Notably, in contrast to Ad, infection was only evident with Ad-gold-PEG when reducing agent BME was used suggesting enhanced selectivity for the tumor environment and therefore safety.

### Example 4: Passive and active targeting of Ad-gold-PEG in vivo

Experiments were performed to test whether the enhanced passive targeting of Ad, achieved as a result of improved stealthing with gold-PEG, could be combined with the increased ultrasound-mediated active targeting, achieved as a result of the increased density provided by stealthing with gold-PEG.

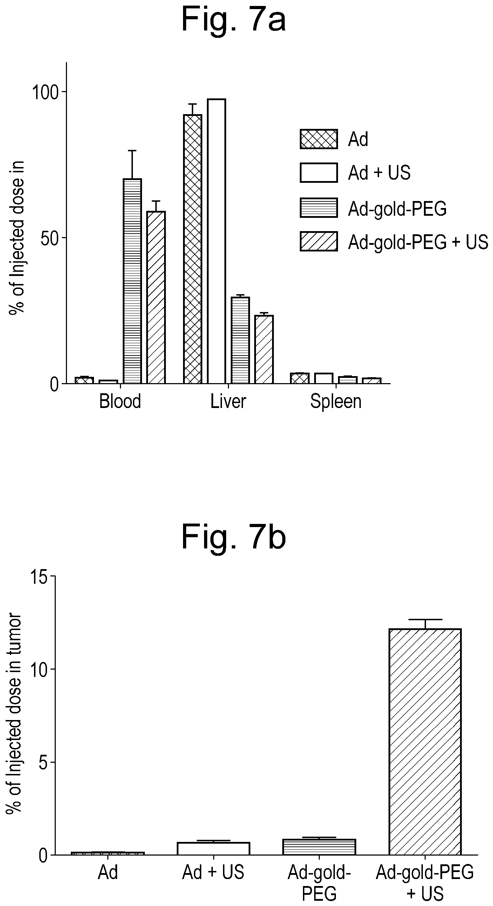

When cancer cell killing oncolytic adenovirus was modified with gold-PEG and delivered to pre-clinical models, in accordance with Fig. 5a, substantially reduced liver capture (29.3%, SD 2.14 vs 91.6% SD 8.36) was obtained, resulting in 35-fold increase in the circulating dose at 30 min (Fig. 7a). This again provided a significant (p<0.005) increase in tumor load of Ad-gold-PEG vs Ad, via passive targeting (0.84% vs 0.12%). When ultrasound was added as a stimulus for active targeting of Ad-gold-PEG a significant (p<0.001) and substantial (14-fold) increase in its tumor accumulation was observed (12.2%, SD 0.97). The increased tumor uptake was even evidenced by a decrease in the amount of dose captured by the liver (23%, SD 1.8).

The combined benefit of improved passive targeting, achieved by enhancing stealthing, and improved ultrasound-mediated active targeting, by enhancing particle density, provided 100-fold more Ad-gold-PEG within ultrasound treated tumors than Ad in non-ultrasound treated tumors.

## Claims

1. A shielded biologic therapeutic comprising a biologic therapeutic which is covalently and cleavably bound to a plurality of nanoparticles, each nanoparticle having a plurality of polymer chains bound thereto, wherein the biologic therapeutic is covalently bound to each nanoparticle via a polymer chain, each polymer chain comprising one or more cleavable moieties.

2. A shielded biologic therapeutic according to claim 1 wherein each nanoparticle is from 1 to 100 nm in size, preferably from 1 to 10 nm in size.

3. A shielded biologic therapeutic according to any preceding claim wherein each nanoparticle is a gold nanoparticle.

4. A shielded biologic therapeutic according to any preceding claim wherein at least 50% of the surface of each nanoparticle is modified by attachment to a polymer chain.

5. A shielded biologic therapeutic according to any preceding claim wherein the polymer chains bound to each nanoparticle are PEG polymer chains.

6. A shielded biologic therapeutic according to any preceding claim wherein the polymer chains bound to each nanoparticle comprise a plurality of polymer chains each having a molecular weight MW1 and one or more polymer chains having a molecular weight MW2, wherein MW2 is greater than MW1 and the number of polymer chains having molecular weight MW1 is greater than the number of chains having MW2.

7. A shielded biologic therapeutic according to claim 7 wherein MW1 is from 1 to 3 kD and MW2 is from 4 to 6 kD; and/or wherein 95% to 99.5% of the polymer chains are of MW1 and 5% to 0.5% are of MW2.

8. A shielded biologic therapeutic according to any preceding claim wherein the cleavable moieties are cleavable under reducing conditions, oxidising conditions, basic conditions, acidic conditions, or conditions with raised endopeptidase levels.

9. A shielded biologic therapeutic according to claim 8 wherein the one or more cleavable moieties comprise N-succinimidyl-3-(2-pyridyldithio)propionate (SPDP).

10. A shielded biologic therapeutic according to any preceding claim wherein the biologic therapeutic is a virus, and wherein the shielded biologic therapeutic is preferably covalently and cleavably bound to from 50 to 200 nanoparticles with a plurality of polymer chains bound thereto.

11. A shielded biologic therapeutic according to any one of claims 1 to 9 wherein the biologic therapeutic is an antibody.

12. A shielded biologic therapeutic according to claim 11 comprising 2 to 5 nanoparticles with a plurality of polymer chains bound thereto.

13. A shielded biologic therapeutic as defined in any preceding claim for use in a method of treatment by therapy of a human or animal subject.

14. A shielded biologic therapeutic for use according to claim 13 wherein the method further comprises exposing the subject to ultrasound-induced cavitation.

15. A shielded biologic therapeutic for use according to claim 13 or 14 wherein the method is for the treatment of a tumour.

16. A method of diagnosis of a human or animal subject which comprises administering to the subject a shielded biologic therapeutic as defined in any one of claims 1 to 12.

17. A method according to claim 16, wherein the method further comprises exposing the subject to ultrasound-induced cavitation.

18. A method according to claim 16 or claim 17 which is for the diagnosis of a tumour.

## Patentansprüche

1. Abgeschirmtes biologisches Therapeutikum, das ein biologisches Therapeutikum umfasst, das kovalent und spaltbar an eine Vielzahl von Nanopartikel gebunden ist, wobei jedes Nanopartikel eine Vielzahl von Polymerketten aufweist, die daran gebunden sind, wobei das biologische Therapeutikum über eine Polymerkette kovalent an jedes Nanopartikel gebunden ist, wobei jede Polymerkette eine oder mehrere spaltbare Einheiten umfasst.

2. Abgeschirmtes biologisches Therapeutikum nach Anspruch 1, wobei jedes Nanopartikel von 1 bis 100 nm groß ist, vorzugsweise von 1 bis 10 nm groß ist.

3. Abgeschirmtes biologisches Therapeutikum nach einem der vorhergehenden Ansprüche, wobei jedes Nanopartikel ein Gold-Nanopartikel ist.

4. Abgeschirmtes biologisches Therapeutikum nach einem der vorhergehenden Ansprüche, wobei mindestens 50 % der Oberfläche jedes Nanopartikels durch Bindung an eine Polymerkette modifiziert ist.

5. Abgeschirmtes biologisches Therapeutikum nach einem der vorhergehenden Ansprüche, wobei die Polymerketten, die an jedes Nanopartikel gebunden sind, PEG-Polymerketten sind.

6. Abgeschirmtes biologisches Therapeutikum nach einem der vorhergehenden Ansprüche, wobei die Polymerketten, die an jedes Nanopartikel gebunden sind, eine Vielzahl von Polymerketten, die jeweils ein Molekulargewicht MW1 aufweisen, und eine oder mehrere Polymerketten, die ein Molekulargewicht MW2 aufweisen, umfassen, wobei MW2 größer als MW1 ist und die Anzahl von Polymerketten, die Molekulargewicht MW1 aufweisen, größer als die Anzahl von Ketten, die MW2 aufweisen, ist.

7. Abgeschirmtes biologisches Therapeutikum nach Anspruch 7, wobei MW1 von 1 bis 3 kD ist und MW2 von 4 bis 6 kD ist; und/oder wobei 95 % bis 99,5 % der Polymerketten MW1 aufweisen und 5 % bis 0,5 % MW2 aufweisen.

8. Abgeschirmtes biologisches Therapeutikum nach einem der vorhergehenden Ansprüche, wobei die spaltbaren Einheiten unter reduzierenden Bedingungen, oxidierenden Bedingungen, basischen Bedingungen, sauren Bedingungen oder Bedingungen mit erhöhten Endopeptidase-Spiegeln spaltbar sind.

9. Abgeschirmtes biologisches Therapeutikum nach Anspruch 8, wobei das eine oder die mehreren spaltbaren Einheiten N-Succinimidyl-3-(2-pyridyldithio)propionat (SPDP) umfassen.

10. Abgeschirmtes biologisches Therapeutikum nach einem der vorhergehenden Ansprüche, wobei das biologische Therapeutikum ein Virus ist und wobei das abgeschirmte biologische Therapeutikum vorzugsweise kovalent und spaltbar an 50 bis 200 Nanopartikel mit einer Vielzahl von Polymerketten, die daran gebunden sind, gebunden ist.

11. Abgeschirmtes biologisches Therapeutikum nach einem der Ansprüche 1 bis 9, wobei das biologische Therapeutikum ein Antikörper ist.

12. Abgeschirmtes biologisches Therapeutikum nach Anspruch 11, umfassend 2 bis 5 Nanopartikel mit einer Vielzahl von Polymerketten, die daran gebunden sind.

13. Abgeschirmtes biologisches Therapeutikum nach einem der vorhergehenden Ansprüche zur Verwendung in einem Verfahren der Behandlung durch Therapie eines menschlichen oder tierischen Subjekts.

14. Abgeschirmtes biologisches Therapeutikum nach Anspruch 13, wobei das Verfahren ferner ein Aussetzen des Subjekts gegenüber ultraschallinduzierter Kavitation umfasst.

15. Abgeschirmtes biologisches Therapeutikum nach Anspruch 13 oder 14, wobei das Verfahren für die Behandlung eines Tumors ist.

16. Diagnoseverfahren eines menschlichen oder tierischen Subjekts, das ein Verabreichen eines abgeschirmten biologischen Therapeutikums nach einem der Ansprüche 1 bis 12 an das Subjekt umfasst.

17. Verfahren nach Anspruch 16, wobei das Verfahren ferner ein Aussetzen des Subjekts gegenüber ultraschallinduzierter Kavitation umfasst.

18. Verfahren nach Anspruch 16 oder Anspruch 17, das für die Diagnose eines Tumors ist.

## Revendications

1. Agent thérapeutique biologique armé comprenant un agent thérapeutique biologique qui est lié de façon covalente et clivable à une pluralité de nanoparticules, chaque nanoparticule ayant une pluralité de chaînes de polymère liée à elle, l'agent thérapeutique biologique étant lié de façon covalente à chaque nanoparticule par une chaîne de polymère, chaque chaîne de polymère comprenant au moins une fraction clivable.

2. Agent thérapeutique biologique armé selon la revendication 1, dans lequel chaque nanoparticule a une taille de 1 à 100 nm, de préférence une taille de 1 à 10 nm.

3. Agent thérapeutique biologique armé selon l'une quelconque des revendications précédentes, dans lequel chaque nanoparticule est une nanoparticule d'or.

4. Agent thérapeutique biologique armé selon l'une quelconque des revendications précédentes, dans lequel au moins 50 % de la surface de chaque nanoparticule est modifiée par fixation à une chaîne de polymère.

5. Agent thérapeutique biologique armé selon l'une quelconque des revendications précédentes, dans lequel les chaînes de polymère liées à chaque nanoparticule sont des chaînes de polymère PEG.

6. Agent thérapeutique biologique armé selon l'une quelconque des revendications précédentes, dans lequel les chaînes de polymère liées à chaque nanoparticule comprennent une pluralité de chaînes de polymère ayant chacune un poids moléculaire MW1 et au moins une chaîne de polymère ayant un poids moléculaire MW2, MW2 étant supérieur à MW1 et le nombre de chaînes de polymère ayant le poids moléculaire MW1 est supérieur au nombre de chaînes ayant MW2.

7. Agent thérapeutique biologique armé selon la revendication 7, dans lequel MW1 va de 1 à 3 kD et MW2 va de 4 à 6 kD ; et/ou dans lequel 95 % à 99,5 % des chaînes de polymère sont de MW1 et 5 % à 0,5 % sont de MW2.

8. Agent thérapeutique biologique armé selon l'une quelconque des revendications précédentes, dans lequel les fractions clivables sont clivables dans des condition de réduction, des conditions d'oxydation, des conditions basiques, des conditions acides ou des conditions avec des taux élevés d'endopeptidase.

9. Agent thérapeutique biologique armé selon la revendication 8, dans lequel l'au moins une fraction clivable comprend du 3-(2-pyridyldithio)propionate de N-succinimidyle (SPDP).

10. Agent thérapeutique biologique armé selon l'une quelconque des revendications précédentes, l'agent thérapeutique étant un virus, et l'agent thérapeutique étant de préférence lié de façon covalente et clivable à 50 à 200 nanoparticules avec une pluralité de chaînes de polymère liées à elles.

11. Agent thérapeutique biologique armé selon l'une quelconque des revendications 1 à 9, l'agent thérapeutique étant un anticorps.

12. Agent thérapeutique biologique armé selon la revendication 11, comprenant 2 à 5 nanoparticules avec une pluralité de chaînes de polymère liées à elles.

13. Agent thérapeutique biologique armé tel que défini dans l'une quelconque des revendications précédentes, destiné à une utilisation dans un procédé de traitement par thérapie d'un sujet humain ou animal.

14. Agent thérapeutique biologique armé destiné à une utilisation selon la revendication 13, dans laquelle le procédé comprend en outre l'exposition du sujet à une cavitation induite par ultrasons.

15. Agent thérapeutique biologique armé destiné à une utilisation selon la revendication 13 ou 14, dans laquelle le procédé est destiné au traitement d'une tumeur.

16. Procédé de diagnostic d'un sujet humain ou animal qui comprend l'administration de l'agent thérapeutique biologique armé tel que défini dans l'une quelconque des revendications 1 à 12.

17. Procédé selon la revendication 16, le procédé comprenant en outre l'exposition du sujet à une cavitation induite par ultrasons.

18. Procédé selon la revendication 16 ou la revendication 17, qui est destiné au diagnostic d'une tumeur.
